(19) Europäisches Patentamt
European Patent Office
Office européen des brevets

(11) EP 4 398 368 A1

(12) **EUROPEAN PATENT APPLICATION**
published in accordance with Art. 153(4) EPC

(43) Date of publication:
**10.07.2024 Bulletin 2024/28**

(21) Application number: **22949677.3**

(22) Date of filing: **13.10.2022**

(51) International Patent Classification (IPC):
***H01M 10/0569** (2010.01)*

(86) International application number:
**PCT/CN2022/125067**

(87) International publication number:
**WO 2024/077544 (18.04.2024 Gazette 2024/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Contemporary Amperex Technology Co., Limited**
**Ningde, Fujian 352100 (CN)**

(72) Inventors:
- **WANG, Hansen**
  **Ningde, Fujian 352100 (CN)**
- **LIU, Chengyong**
  **Ningde, Fujian 352100 (CN)**
- **HUANG, Shengyuan**
  **Ningde, Fujian 352100 (CN)**
- **HUANG, Xian**
  **Ningde, Fujian 352100 (CN)**
- **XUE, Wenwen**
  **Ningde, Fujian 352100 (CN)**

(74) Representative: **Jacob, Reuben Ellis et al**
**Maucher Jenkins**
**Seventh Floor Offices**
**Artillery House**
**11-19 Artillery Row**
**London SW1P 1RT (GB)**

# (54) ELECTROLYTE SOLUTION, BATTERY CELL COMPRISING SAME, BATTERY AND ELECTRIC DEVICE

(57) The application provides an electrolyte containing a solvent including at least one compounds of formula I, wherein $A_1$ is an oxygen atom or a single bond, $A_2$ is a single bond or $CHR_4$, $R_1$ and $R_2$ are each independently a hydrogen atom, C1-6 alkyls or a C1-6 fluoroalkyls, $R_3$ and $R_4$ are each independently a hydrogen atom, a fluorine atom, C1-6 alkyls or C1-6 fluoroalkyls, A1 and A2 cannot both be single bonds simultaneously, and only one of $R_1$ to $R_4$ includes a fluorine atom. The electrolyte of the present disclosure can make a metal negative electrode secondary battery have good cycling performance and good discharge capacity retention at low temperature.

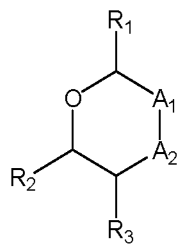

I

EP 4 398 368 A1

## Description

### TECHNICAL FIELD

The present disclosure relates to the technical field of lithium batteries, and more particularly, to an electrolyte, a battery cell, a battery, and an electrical device.

### BACKGROUND

Metal negative secondary battery has much higher volumetric energy density than conventional secondary battery, and thus it has great application potential in high-endurance electric vehicles, unmanned aerial vehicles, electric aircraft and other application scenarios. However, such batteries also have disadvantages such as poor circulation ability, poor low-temperature capacity release ability, and the like, as compared with the conventional secondary battery.
There is a need in the art for an electrolyte that can improve the cycling performance of a metal negative secondary battery and improve its low temperature performance at the same time.

### SUMMARY

The present disclosure has been made in view of the above-mentioned problems, and an object thereof is to provide an electrolyte, which enables a metal negative secondary battery to have an excellent cycle life and a significantly improved low-temperature capacity release capability.
A first aspect of the present disclosure provides an electrolyte, containing a solvent, including at least one compound of formula I:

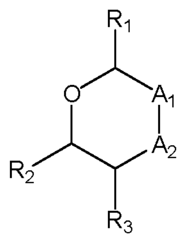

I

where

- $A_1$ is an oxygen atom or a single bond;
- $A_2$ is a single bond or $CHR_4$;
- $R_1$ and $R_2$ are each independently a hydrogen atom, C1-C6 alkyls or C1-C6 fluoroalkyls;
- $R_3$ and $R_4$ are each independently a hydrogen atom, a fluorine atom, C1-C6 alkyls or C1-C6 fluoroalkyls;
- $A_1$ and $A_2$ cannot be single bonds simultaneously, and
- only one of $R_1$ to $R_4$ includes a fluorine atom.

The electrolyte of the present disclosure enables a metal negative secondary battery to have good cycling performance and good discharge capacity retention rate at low temperatures over a wide temperature range.
In any embodiment, $R_1$ and $R_2$ are each independently a hydrogen atom, C1-C4 alkyls or C1-C4 fluoroalkyls; optionally, $R_1$ and $R_2$ are each independently a hydrogen atom, a methyl, or a fluoromethyl; more optionally, $R_1$ and $R_2$ are each independently a hydrogen atom or a fluoromethyl; still more optionally, $R_1$ and $R_2$ are each independently a hydrogen atom.
In any embodiment, $R_3$ and $R_4$ are each independently a hydrogen atom, a fluorine atom, C1- C 4 alkyls and C1- C4 fluoroalkyls; optionally, $R_3$ and $R_4$ are each independently a hydrogen atom, a fluorine atom, a methyl, or a fluoromethyl.
In any embodiment, $R_3$ is a fluorine atom or a hydrogen atom, optionally a fluorine atom.
In any embodiment, in each formula, $R_4$ is a hydrogen atom.
Further selecting the respective groups in the above-mentioned compound of formula I make the electrolyte of the present disclosure have good oxidation resistance, can effectively dissociate the electrolyte salts, and is more conducive to improving the cycling performance of the secondary battery over a wide temperature range and having a desirable discharge capacity at a low temperature.
In any embodiment, the compound of formula I is selected from at least one of the following compounds:

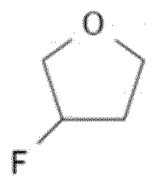
I-1 ,
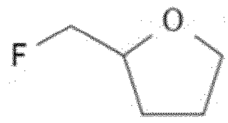
I-9 ,
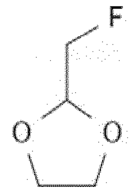
I-57 , and
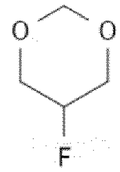
I-78 .

The inclusion of the above-mentioned compounds in the electrolyte of the present disclosure can be more beneficial to improve the cycling performance and low-temperature discharge capacity retention rate of the secondary battery.

In any embodiment, the solvent further comprises one or more selected from: ethylene glycol dimethyl ether, ethylene glycol methyl ethyl ether, ethylene glycol diethyl ether, ethylene glycol dipropyl ether, diethylene glycol dimethyl ether, triethylene glycol dimethyl ether, tetraethylene glycol dimethyl ether, propylene glycol dimethyl ether, butylene glycol dimethyl ether, dimethoxymethane, diethoxymethane, trimethyl orthoformate, triethyl orthoformate, trimethyl orthocarbonate, triethyl orthocarbonate, ethylene carbonate, vinylene carbonate, dimethyl carbonate, methyl ethyl carbonate, diethyl carbonate, fluoroethylene carbonate, acetonitrile, dimethyl sulfoxide, sulfolane, bis(2,2,2-trifluoroethyl) ether, 1,1,2,2-tetrafluoroethyl-2,2,3,3-tetrafluoropropyl ether, 1,2-bis(1,1,2,2-tetrafluoroethoxy) ethane, fluorobenzene, p-difluorobenzene, o-difluorobenzene, m-difluorobenzene, trifluorobenzene, trifluorotoluene, trifluoromethoxybenzene, acetone and perfluoropentanone; optionally, the solvent further comprises dimethyl carbonate and/or perfluoropentanone.

In any embodiment, the solvent includes at least 20 wt%, more optionally at least 60 wt% of the compound of formula I, based on a total weight of the solvent. Using the compound of formula I with the above-mentioned percentages in the electrolyte can achieve high oxidation resistance and high ion transport properties at the same time.

In any embodiment, the electrolyte further includes an additive selected from at least one of: succinic anhydride, vinylene carbonate, 1,3-propane sultone, ethylene vinyl carbonate, fluoroethylene carbonate, vinyl sulfite, tris(trimethylsilane) phosphate.

In any embodiment, a content of the additive is 5wt% or less based on the total weight of the electrolyte.

The use of the additives is conducive to further improving the battery performance.

In any embodiment, the electrolyte further includes an electrolyte salt selected from at least one of lithium bis-fluorosulfonimide, lithium hexafluorophosphate, lithium tetrafluoroborate, lithium hexafluoroarsenate, lithium bis-trifluoromethane sulfonimide, lithium triflate, lithium difluorophosphate, lithium bis(oxalate) borate, lithium difluorooxalato borate, lithium bis(oxalate) phosphate, and lithium tetrafluorooxalato phosphate; optionally, at least one of lithium difluorosulfonamide, lithium hexafluorophosphate, and lithium tetrafluoroborate; more optionally, lithium bis-fluorosulfonimide. The use of the above-mentioned electrolyte salt is more conducive to improving the cycling performance of the metal negative secondary battery and improving the discharge capacity retention of the battery under low-temperature conditions.

In any embodiment, the concentration of the electrolyte salt is 0.5 M to 5 M, optionally 1 M to 3 M, more optionally 1.5 M to 2.5 M. Controlling the electrolyte salt concentration in the above-mentioned range is more conducive to improving the cycling performance and low-temperature performance of the secondary battery.

A second aspect of the present disclosure provides a battery cell including the electrolyte of the first aspect of the present disclosure.

In any embodiment, the battery cell further includes a negative electrode plate comprising a current collector and optionally a layer of negative material disposed on at least one surface of the current collector, wherein the negative material is a lithium-containing metal; optionally, the lithium-containing metal is simple lithium metal or a lithium-containing alloy; the lithium-containing alloy is an alloy formed by lithium and other metallic elements or non-metallic elements, wherein the metallic elements include tin (Sn), zinc (Zn), aluminum (Al), magnesium (Mg), silver (Ag), gold (Au), gallium (Ga), indium (In), and platinum (Pt), and combinations thereof; the non-metallic elements include boron (B), carbon (C), and silicon (Si) and combinations thereof.

A third aspect of the present disclosure provides a battery including the battery cell of the second aspect of the present disclosure.

A fourth aspect of the present disclosure provides an electrical device, including the battery cell of the second aspect and/or the battery of the third aspect.

The electrolyte of the present disclosure enables a metal negative secondary battery to have an excellent cycle life and a significantly improved low-temperature capacity release capability.

## BRIEF DESCRIPTION OF DRAWINGS

Fig. 1 is a schematic diagram of a secondary battery of an embodiment of the present disclosure.
Fig. 2 is an exploded diagram of the secondary battery of an embodiment of the present disclosure as shown in Fig. 1.
Fig. 3 is a schematic diagram of a battery module of an embodiment of the present disclosure.
Fig. 4 is a schematic diagram of a battery pack of an embodiment of the present disclosure.
Fig. 5 is an exploded diagram of the battery pack of an embodiment of the present disclosure as shown in Fig. 4.
Fig. 6 is a schematic diagram of an electrical device in which a secondary battery is used as a power source according to an embodiment of the present disclosure.

Reference numerals:
1 battery pack; 2 upper box; 3 lower box; 4 battery module; 5 secondary battery; 51 housing; 52 electrode assembly; 53 top cover assembly

## DESCRIPTION OF EMBODIMENTS

Hereinafter, embodiments of an electrolyte, a secondary battery, a battery module, a battery pack, and an electrical device of the present disclosure are specifically disclosed with reference to the accompanying drawings as appropriate. However, there may be cases where unnecessary detailed description is omitted. For example, detailed descriptions of well-known matters and repeated descriptions of practically identical structures are omitted. This is to avoid the following description from becoming unnecessarily lengthy and to facilitate the understanding of those skilled in the art. In addition, the drawings and the following description are provided to enable those skilled in the art to fully understand the present disclosure and are not intended to limit the subject matter recited in the claims.

The "ranges" disclosed herein are defined in terms of lower and upper limits, a given range is defined by selecting a lower limit and an upper limit, and the selected lower and upper limits define the boundaries of the particular range. Ranges defined in this manner may or may not be inclusive of the end values and can be arbitrarily combined, i.e. any lower limit can be combined with any upper limit to form a range. For example, if ranges of 60-120 and 80-110 are listed for a particular parameter, it is understood that ranges of 60-110 and 80-120 are also contemplated. In addition, if the minimum range values listed are 1 and 2, and if the maximum range values listed are 3, 4, and 5, the following ranges are all contemplated: 1-3, 1-4, 1-5, 2-3, 2-4 and 2-5. In the present disclosure, unless otherwise indicated, the numerical range "a-b" represents an abbreviated representation of any real number combination between a and b, where a and b are both real numbers. For example, a numerical range of "0-5" indicates that all real numbers between "0-5" have been fully set forth herein, and "0-5" is merely a shorthand representation of combinations of these numbers. In addition, when it is stated that a certain parameter is an integer of $\geq 2$, it is equivalent to disclosing that the parameter is, for example, an integer of 2, 3, 4, 5, 6, 7, 8, 9, 10, 11, 12, etc.

All embodiments and alternative embodiments of the present disclosure may be combined with each other to form a new technical solution if not specifically stated.

All technical features and alternative technical features of the present disclosure may be combined with each other to form a new technical solution if not specifically stated.

Unless otherwise specified, all steps of the present disclosure may be performed sequentially or may be performed randomly, preferably sequentially. For example, the process comprises steps (a) and (b), meaning that the process may comprise steps (a) and (b) performed sequentially, or may comprise steps (b) and (a) performed sequentially. For example, the reference to the process may further comprise step (c), meaning that step (c) may be added to the process in any order. For example, the process may comprise steps (a), (b) and (c), may also comprise steps (a), (c) and (b), may also comprise steps (c), (a) and (b), etc.

Unless otherwise specified, references to "comprising" and "containing" in the present disclosure are intended to be open-ended as well as closed-ended. For example, reference to "comprising" and "containing" may mean that other components not listed may also be comprised or contained, or that only listed components may be comprised or contained.

In the present disclosure, the term "or" is inclusive if not specifically stated. For example, the phrase "A or B" means "A, B, or both A and B". More specifically, condition "A or B" is satisfied by any one of the following conditions: A is true (or present) and B is false (or not present); A is false (or absent) and B is true (or present); or both A and B are true (or present).

Metal negative secondary battery has much higher volumetric energy density than conventional secondary battery, and thus it has great application potential in high-endurance electric vehicles, unmanned aerial vehicles, electric aircraft and other application scenarios. However, such batteries also have disadvantages such as poor circulation ability

and the like, as compared with the conventional secondary battery.

In order to improve the cycling performance of a metal negative electrode secondary battery (e.g. a lithium metal battery or a sodium metal battery), those skilled in the art have made many efforts in which improvement of an electrolyte is an important aspect. Although the normal-temperature cycling performance of the metal negative secondary battery is improved through the optimization and development of the electrolyte formulation, it is still difficult to maintain good performance over a wide range of working temperature range, and particularly the performance (e.g. cycle life at low temperatures, capacity release ability) of the battery under extreme conditions (especially at low temperatures) is still unsatisfactory.

Currently, the chain ether-based electrolytes commonly used in metal negative secondary batteries are often accompanied by problems of high concentration (i.e. high electrolyte salt concentration), high viscosity, low ionic conductivity, etc. It is very challenging to ensure low-temperature capacity release and long cycle life. Cyclic ether species (e.g. epoxy compounds, particularly tetrahydrofuran and its derivative compounds) have properties that may be advantageous for improving low temperature performance, such as low boiling and freezing points, low viscosity, etc. but such species often have poor oxidation stability, which is not conducive to being used as the primary solvent in an electrolyte.

In view of the problems, there is a need in the art for an electrolyte that can improve both the normal temperature and low temperature performance of a metal negative secondary battery.

An object of the present disclosure is to provide an electrolyte that enables a metal negative secondary battery to have excellent performance (e.g. long cycle life and high capacity release at low temperatures) over a wide temperature range.

**Electrolyte**

An aspect of the present disclosure provides an electrolyte, containing a solvent including at least one compound of formula I:

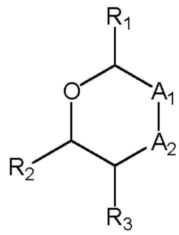

I

where

$A_1$ is an oxygen atom or a single bond;
$A_2$ is a single bond or $CHR_4$;
$R_1$ and $R_2$ are each independently a hydrogen atom, C1-C6 alkyls or C1-C6 fluoroalkyls;
$R_3$ and $R_4$ are each independently a hydrogen atom, a fluorine atom, C1-C6 alkyls or C1-C6 fluoroalkyls;
$A_1$ and $A_2$ cannot be single bonds simultaneously, and
only one of $R_1$ to $R_4$ includes a fluorine atom.

The electrolyte of the present disclosure enables a metal negative electrode secondary battery to have good normal temperature and low temperature cycling performance and have good discharge capacity retention at low temperature.

Herein, "single bond" has the meaning commonly understood by those skilled in the art and means a covalent bond formed by sharing a pair of electrons between two atoms, usually indicated by a short line "-".

Herein, "discharge capacity retention rate" is defined as the average capacity release of a battery at 25 °C as $C_0$, and the cycle average capacity release at t°C as Ct, then the capacity retention is $C_t/C_0$.

Herein, "solvent" has the commonly understood meaning, i.e. a liquid used to dissolve a solute; specifically, in the present disclosure, the solvent is a liquid compound or a mixture thereof for dissolving an electrolyte salt, an additive (if present) and the like contained in an electrolytic solution, and usually occupies an amount of, for example, at least 10% by weight in the electrolyte.

Herein, "low temperature" means a temperature ranging from about -40°C to about 0°C.

Without wishing to be bound by any theory, the inventors have found that the compounds of formula I of the present disclosure have a 5-or 6-membered ring structure, making them less susceptible to side reactions which will deteriorate the battery performance such as oxidative ring opening, polymerization, etc. during the cycling of the battery

due to moderate ring tension and structural stability. At the same time, such a ring structure also enables the compound to have appropriate solvation ability, which is beneficial to maintain a desired discharge capacity of the secondary battery at a lower temperature. When the compound has a ring structure of less than 5-membered (e.g. 3-membered or 4-membered), the ring tension is large, unstable and easily decomposed, while a ring structure of more than 6-membered (e.g. 7-membered and above) has a large volume and poor ion transport property. Thus, they are not an ideal candidate for a solvent. Meanwhile, for the present disclosure, it is advantageous that the substituents of the compound of formula I is are substituents with small volume, for example, a hydrogen atom, a fluorine atom, C1- C6 alkyls or C1- C6 fluoroalkyls, and such substituents with small volume make the compound and the solvent containing the compound have a lower melting boiling point and viscosity, facilitating the maintenance of the discharge capacity of the battery at a low temperature. In addition, the fluorine-containing substituents (including fluoroalkyls and the fluorine atom) in the compound of formula I are necessary to achieve the desired effects of the present disclosure, and such substituents make use of the strong electron-withdrawing effect of the fluorine atom to impart improved oxidation resistance to the compound, which is more favorable for achieving a long-term stable cycle of the metal negative electrode secondary battery. However, at the same time, the inventors have also found that in the compound of formula I of the present disclosure, there is no more than one fluorine-containing substituent, and the fluorine atom cannot be directly substituted on the carbon atom adjacent to the oxygen atom in the ring structure, because the strong electron-withdrawing effect of the fluorine atom causes strong attraction to the lone pair of electrons of the oxygen atom, and therefore a reasonable quantity and position of the fluorine-containing substituents are more conducive to maintaining the ability of the oxygen atom to bind to lithium ions or sodium ions, so that solvent molecules can effectively dissociate (or solvate) electrolyte salts, resulting in a higher ion conductivity in the electrolyte system, thereby improving the low-temperature discharge performance thereof.

In some embodiments, $R_1$ and $R_2$ are each independently a hydrogen atom, C1-C4 alkyls or C1-C4 fluoroalkyls. R1 or R2 is advantageously selected from the group consisting of a hydrogen atom and alkyls or haloalkyls with shorter carbon chains, as such compounds of formula I may have desirably lower melting and boiling points and viscosities, thereby facilitating maintenance of the capacity release capability of the battery at lower temperatures. optionally, the fluoroalkyl is a monofluoroalkyl. In some embodiments, $R_1$ and $R_2$ are each independently a hydrogen atom, a methyl, or a fluoromethyl. Optionally, the fluoromethyl is monofluoromethyl. In some embodiments, $R_1$ and $R_2$ are each independently a hydrogen atom or fluoromethyls. In some embodiments, $R_1$ and $R_2$ are each independently a hydrogen atom.

In some embodiments, $R_3$ and $R_4$ are each independently a hydrogen atom, a fluorine atom, C1-C4 alkyls or C1-C4 fluoroalkyls. $R_3$ or $R_4$ is advantageously selected from the group consisting of a hydrogen atom, a fluorine atom and alkyls or haloalkyls with shorter carbon chains, as such compounds of formula I may have desirably lower melting and boiling points and viscosities, thereby facilitating maintenance of the capacity release capability of the battery at lower temperatures. Optionally, the fluoroalkyl is a monofluoroalkyl. In some embodiments, $R_3$ and $R_4$ are each independently a hydrogen atom, a fluorine atom, a methyl, or a fluoromethyl. Optionally, the fluoromethyl is monofluoromethyl.

In some embodiments, $R_3$ is a fluorine atom or a hydrogen atom; optionally, $R_3$ is a fluorine atom.

In some embodiments, $R_4$ is a hydrogen atom.

Further selecting the above-mentioned compound of formula I having the above-mentioned substituents can make the electrolyte of the present disclosure have good oxidation resistance, can effectively dissociate the electrolyte salt, and is more conductive to improving the cycling performance of the secondary battery over a wide temperature range and having a desirable discharge capacity at a low temperature.

In some embodiments, the solvent includes a compound of formula I selected from one or more of the following compounds:

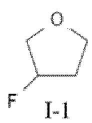 I-1
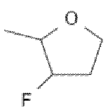 I-2
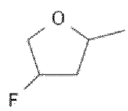 I-3
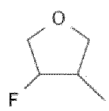 I-4
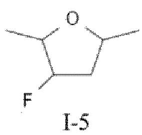 I-5
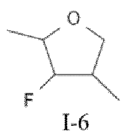 I-6

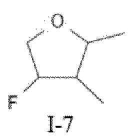 I-7
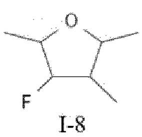 I-8
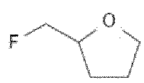 I-9
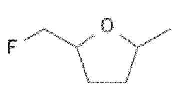 I-10
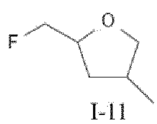 I-11
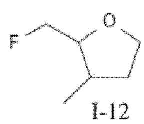 I-12

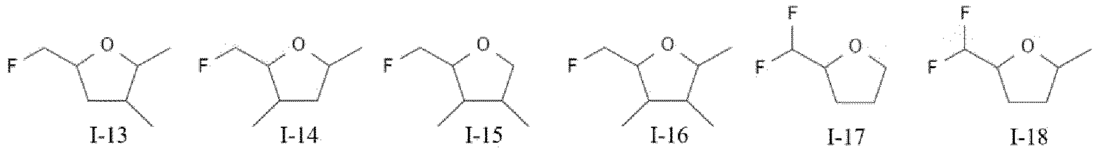
I-13 I-14 I-15 I-16 I-17 I-18

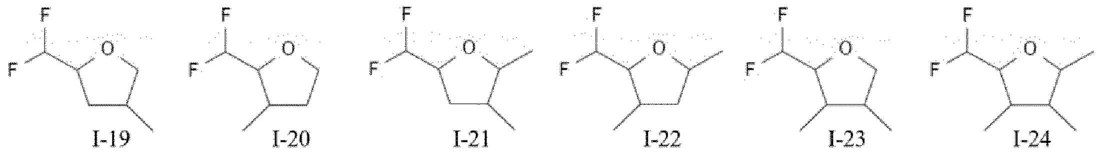
I-19 I-20 I-21 I-22 I-23 I-24

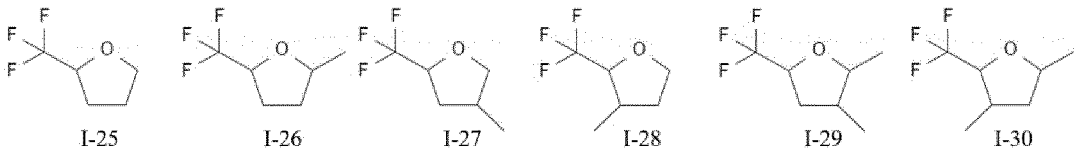
I-25 I-26 I-27 I-28 I-29 I-30

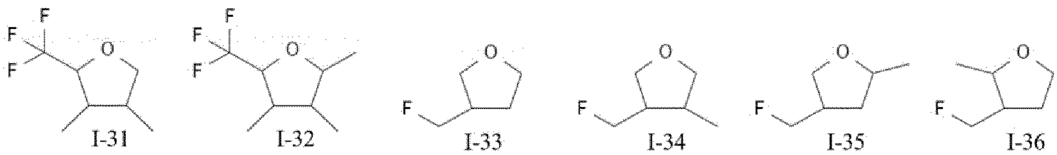
I-31 I-32 I-33 I-34 I-35 I-36

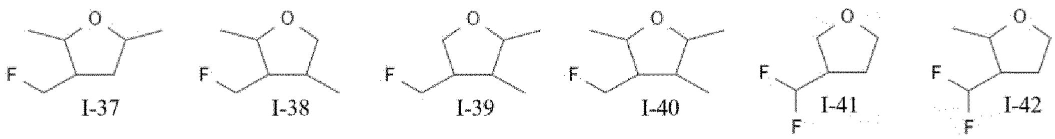
I-37 I-38 I-39 I-40 I-41 I-42

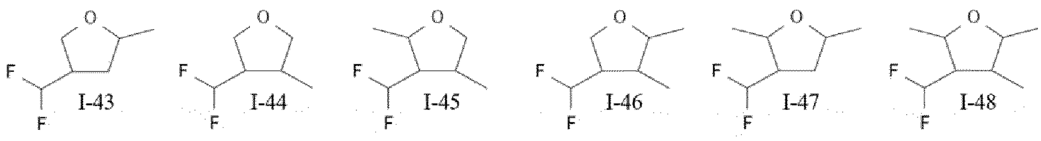
I-43 I-44 I-45 I-46 I-47 I-48

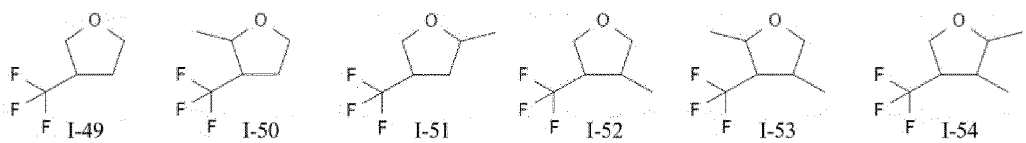
I-49 I-50 I-51 I-52 I-53 I-54

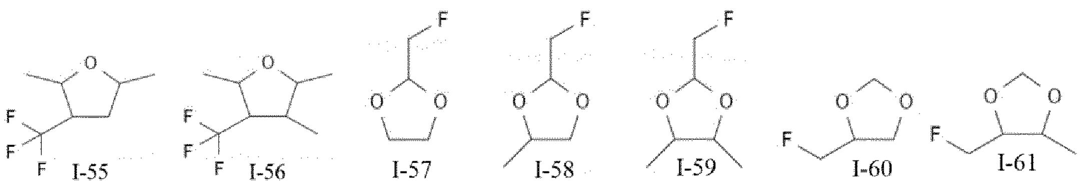
I-55 I-56 I-57 I-58 I-59 I-60 I-61

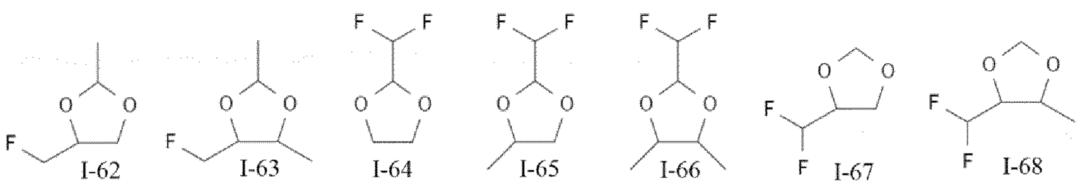
I-62 I-63 I-64 I-65 I-66 I-67 I-68

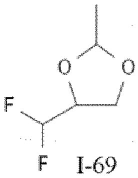
I-69

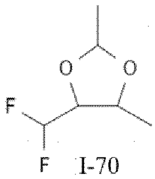
I-70

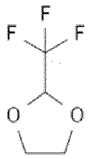
I-71

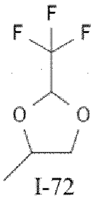
I-72

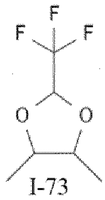
I-73

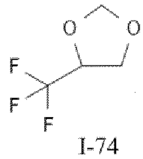
I-74

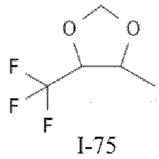
I-75

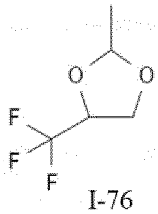
I-76

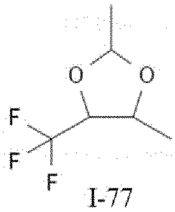
I-77

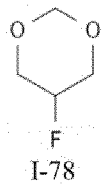
I-78

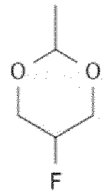
I-79

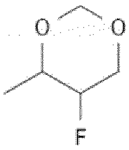
I-80

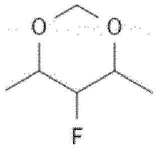
I-81

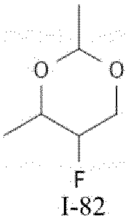
I-82

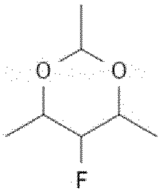
I-83

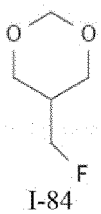
I-84

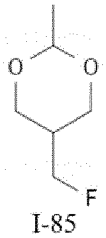
I-85

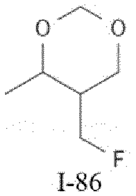
I-86

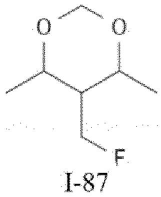
I-87

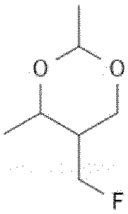
I-88

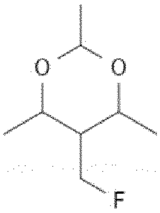
I-89

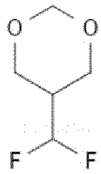
I-90

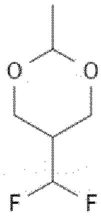
I-91

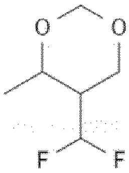
I-92

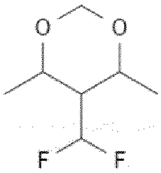
I-93

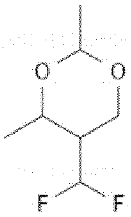
I-94

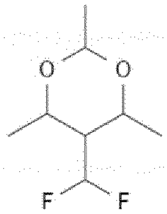
I-95

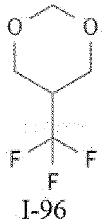
I-96

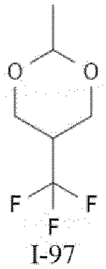
I-97

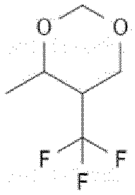
I-98

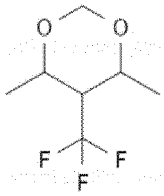
I-99

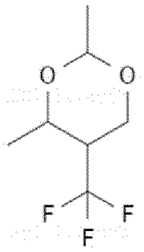
I-100

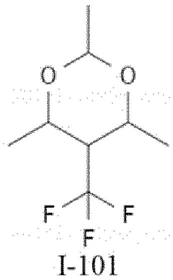
I-101

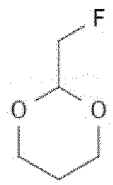
I-102

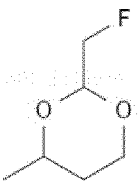
I-103

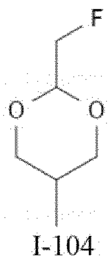
I-104

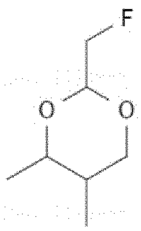
I-105

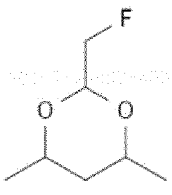
I-106

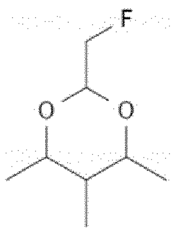
I-107

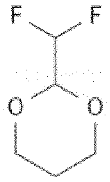
I-108

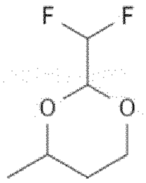
I-109

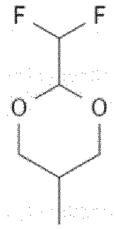
I-110

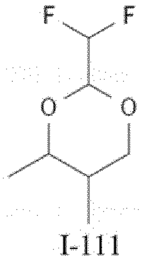
I-111

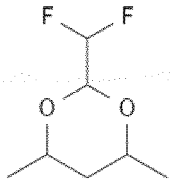
I-112

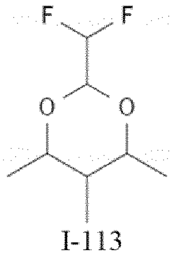
I-113

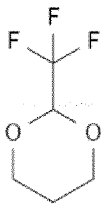
I-114

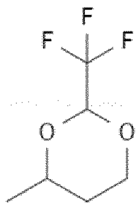
I-115

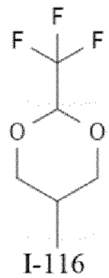
I-116

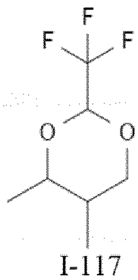
I-117

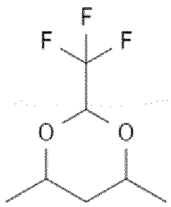
I-118

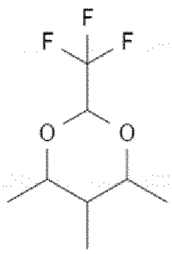
I-119

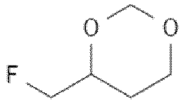
I-120

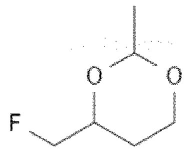
I-121

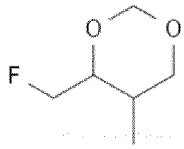
I-122

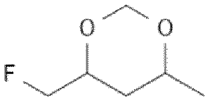
I-123

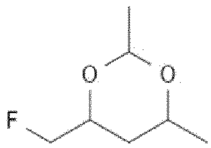
I-124

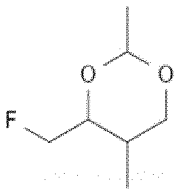
I-125

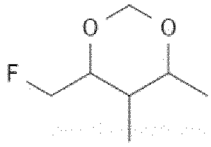
I-126

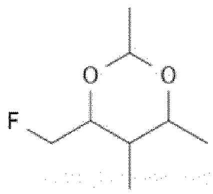
I-127

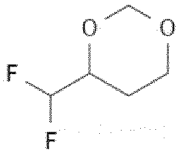
I-128

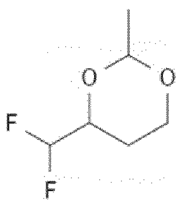
I-129

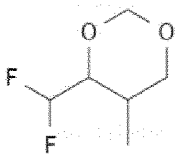
I-130

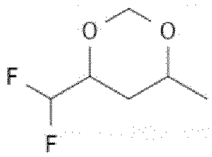
I-131

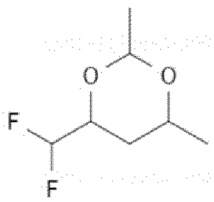
I-132

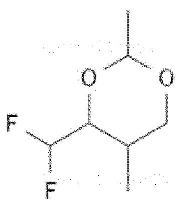
I-133

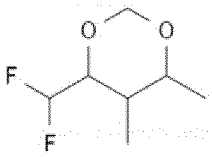
I-134

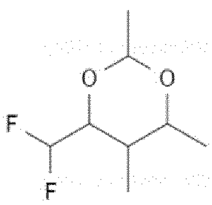
I-135

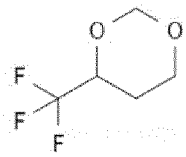
I-136

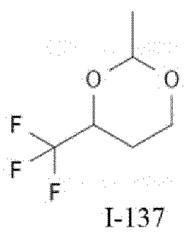
I-137

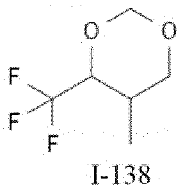
I-138

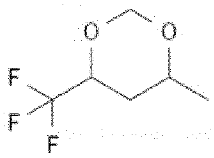
I-139

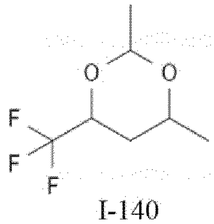
I-140

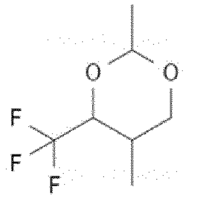
I-141

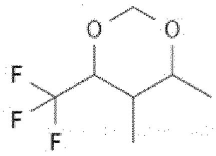
I-142

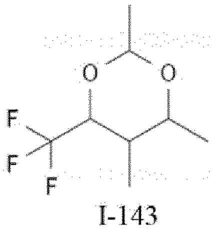
I-143

In some embodiments, the solvent includes at least one of compounds 1-1, 1-9, 1-57, and 1-78. In some embodiments, the solvent includes at least one of compounds 1-1 and 1-78; more optionally, the solvent includes compound 1-1. In some embodiments, the solvent includes one or more of the preferred compounds described above. The use of the above-mentioned compound of formula I in the electrolyte of the present disclosure further improves the cycling performance and low-temperature discharge capacity retention rate of the secondary battery due to its good oxidation resistance and electrolyte salt dissociation capability at the same time.

In some embodiments, in the electrolyte the solvent further includes one or more selected from: ethylene glycol dimethyl ether, ethylene glycol methyl ethyl ether, ethylene glycol diethyl ether, ethylene glycol dipropyl ether, diethylene glycol dimethyl ether, triethylene glycol dimethyl ether, tetraethylene glycol dimethyl ether, propylene glycol dimethyl ether, butylene glycol dimethyl ether, dimethoxymethane, diethoxymethane, trimethyl orthoformate, triethyl orthoformate, trimethyl orthocarbonate, triethyl orthocarbonate, ethylene carbonate, vinylene carbonate, dimethyl carbonate, methyl ethyl carbonate, diethyl carbonate, fluoroethylene carbonate, acetonitrile, dimethyl sulfoxide, sulfolane, bis(2,2,2-trifluoroethyl) ether, 1,1,2,2-tetrafluoroethyl-2,2,3,3-tetrafluoropropyl ether, 1,2-bis(1,1,2,2-tetrafluoroethoxy) ethane, fluorobenzene, p-difluorobenzene, o-difluorobenzene, m-difluorobenzene, trifluorobenzene, trifluorotoluene, trifluoromethoxybenzene, acetone and perfluoropentanone. In some embodiments, optionally, the solvent further includes dimethyl carbonate and/or perfluoropentanone. These solvents above have relatively low melting points, good miscibility with the compounds of formula I of the present disclosure and excellent stability to positive and negative electrodes; it can be added thereto to reduce the cost without significantly losing the excellent performance of the battery.

In some embodiments, the solvent includes at least 20 wt%, more optionally at least 60 wt% of the compound of formula I, based on a total weight of the solvent. The inclusion of a compound of formula I in the above-mentioned range in the electrolyte can achieve high oxidation resistance, low viscosity and high ion transport properties at the same time. In some embodiments, the solvent includes at least 60 wt% of the compound of formula I, based on a total weight of the solvent.

In some embodiments, the electrolyte further includes an electrolyte salt, wherein the electrolyte salt is selected from at least one of lithium bis-fluorosulfonimide, lithium hexafluorophosphate, lithium tetrafluoroborate, lithium hexafluoroarsenate, lithium bis-trifluoromethane sulfonimide, lithium triflate, lithium difluorophosphate, lithium bis(oxalato) borate, lithium difluorooxalato borate, lithium difluoro bis(oxalato) phosphate, and lithium tetrafluorooxalato phosphate; optionally, the electrolyte salt is selected from at least one of lithium difluorosulfonamide, lithium hexafluorophosphate, and lithium tetrafluoroborate; more optionally, the fluorine-containing electrolyte salt is lithium bis-fluorosulfonimide. The above-mentioned electrolyte salts can decompose on the surface of the metal negative electrode to form SEI components rich in inorganic fluorine, which is more conducive to improving the cycling performance of the metal negative electrode battery; at the same time, the combination of the above-mentioned electrolyte salt with the compound of formula (I) can impart a higher ionic conductivity and a lower viscosity to the electrolyte, which is further conducive to improving the discharge capacity retention capacity of the secondary battery under low-temperature conditions. Lithium bis-fluorosulfonimide is particularly optional, which can significantly improve the performance of the electrolyte, particularly low temperature performance.

In some embodiments, the electrolyte salt can also be one or more of sodium hexafluorophosphate ($NaPF_6$), sodium hexafluoroborate ($NaBF_4$), $NaN(SO_2F)_2$ (abbreviated as NaFSI), $NaClO_4$, $NaAsF_6$, $NaB(C_2O_4)_2$ (abbreviated as NaBOB), $NaBF_2(C_2O_4)$ (abbreviated as NaDFOB), $NaN(SO_2R_F)_2$, and $NaN(SO_2F)(SO_2R_F)$, wherein $R_F$ represents $C_bF_{2b+1}$ and b is an integer in the range of 1-10, optionally in the range of 1 to 3, more optionally $R_F$ is $-CF_3$, $-C_2F_5$ or

$-CF_2CF_2CF_3$.

In some embodiments, the concentration of the electrolyte salt is 0.5 M to 5 M, optionally 1 M to 3 M, more optionally 1.5 M to 2.5 M. In some embodiments, the concentration of the electrolyte salt is 2M. Controlling the electrolyte salt concentration in the above-mentioned range can make the electrolyte have better ion transport capacity and more desirable viscosity, which is conducive to improving the interfacial stability of the plates, and further improving the cycling performance and low-temperature performance.

In some embodiments, the electrolyte of the present disclosure also optionally incudes an addictive. For example, the additive may include a negative film-forming additive, a positive film-forming additive, and may further include an additive capable of improving certain properties of the battery, such as an additive for improving overcharge properties of the battery, an additive for improving high-temperature or low-temperature properties of the battery, etc.

In some embodiments, the additive is selected from at least one of: succinic anhydride, vinylene carbonate, 1,3-propane sultone, ethylene vinyl carbonate, fluoroethylene carbonate, vinyl sulfite, tris(trimethylsilane) phosphate.

In some embodiments, a content of the additive is 5wt% or less based on the total weight of the electrolyte.

The use of the additives is conducive to further improving the battery performance.

In some embodiments, the electrolyte of the present disclosure is used for lithium metal secondary batteries or sodium metal secondary batteries, optionally for lithium metal secondary batteries.

**Battery cell, battery, and electrical device**

Another aspect of the present disclosure provides a battery cell including the electrolyte described above.

Herein, "battery cell" and "secondary battery" have the same or similar meanings.

In some embodiments, the battery cell of the present disclosure is selected from lithium metal secondary battery cell or sodium metal secondary battery cell.

In some embodiments, the battery cell of the present disclosure further includes a negative electrode plate including a current collector and a layer of negative material disposed on at least one surface of the current collector, wherein the negative material is a lithium-containing or sodium-containing metal; optionally, the negative material is a lithium-containing metal; optionally, the lithium-containing metal is simple lithium metal or a lithium-containing alloy. In some embodiments, optionally, the lithium-containing alloy is an alloy formed by lithium and other metallic elements or non-metallic elements, wherein the metallic elements include tin (Sn), zinc (Zn), aluminum (Al), magnesium (Mg), silver (Ag), gold (Au), gallium (Ga), indium (In), and platinum (Pt), and combinations thereof; the non-metallic elements include boron (B), carbon (C), and silicon (Si) and combinations thereof.

A third aspect of the present disclosure provides a battery pack including the battery cell of the present disclosure. The battery of the present disclosure may be, for example, a battery module or a battery pack.

A fifth aspect of the present disclosure provides an electrical device including the battery cell selected from the present disclosure and/or the battery of the present disclosure.

In addition, the battery cell, battery (including, for example, battery module, battery pack), and electrical device of the present disclosure will be explained with appropriate reference to the attached drawings.

An embodiment of the present disclosure provides a battery cell.

Generally, the battery cell includes a positive electrode plate, a negative electrode plate, an electrolyte, and a separator. During charging and discharging of the battery, active ions are intercalated and deintercalated between the positive electrode plate and the negative electrode plate. The electrolyte serves to conduct ions between the positive electrode plate and the negative electrode plate. The separator is arranged between the positive electrode plate and the negative electrode plate, and mainly serves to prevent short circuit between the positive and negative electrodes, and at the same time it can enable the ions to pass through. The reactive ions are selected from the group consisting of lithium ions and sodium ions.

[Positive electrode plate]

The positive electrode plate typically includes a positive electrode current collector and a positive film layer disposed on at least one surface of the positive electrode current collector, the positive film layer includes a positive electrode active material.

As an example, the positive electrode current collector has two surfaces opposed in its own thickness direction, and a positive film layer is provided on either or both of the two surfaces opposed to the positive electrode current collector.

In some embodiments, the positive electrode current collector may be a metal foil or a composite current collector. For example, aluminum foil may be used as the metal foil. The composite current collector may include a polymer material base layer and a metal layer formed on at least one surface of the polymer material base layer. The composite current collector may be formed by forming a metallic material (aluminum, aluminum alloy, nickel, nickel alloy, titanium, titanium alloy, silver, silver alloy, etc.) on a substrate of a high molecular material such as a substrate of

polypropylene (PP), polyethylene terephthalate (PET), polybutylene terephthalate (PBT), polystyrene (PS), polyethylene (PE), etc.

In some embodiments, the positive electrode material may use, but is not limited to, a lithium transition metal oxide and/or a lithium phosphate of an olivine structure, a sodium transition metal oxide, a polyanionic compound, and a Prussian blue-based compound as the positive electrode active material.

In some embodiments, the positive electrode active material is selected from lithium transition metal oxides. In some embodiments, the lithium transition metal oxide is selected from lithium cobalt oxide, lithium nickel oxide, lithium manganese oxide, lithium nickel manganese oxide, lithium nickel cobalt manganese oxide, lithium nickel cobalt aluminum oxide, or a combination thereof.

Examples of lithium transition metal oxides can include, but are not limited to, at least one of lithium cobalt oxides (e.g. $LiCoO_2$), lithium nickel oxides (e.g. $LiNiO_2$), lithium manganese oxides (e.g. $LiMnO_2$, $LiMn_2O_4$), lithium nickel cobalt oxides, lithium manganese cobalt oxides, lithium nickel manganese oxides, lithium nickel cobalt manganese oxides (e.g. $LiNi_{1/3}Co_{1/3}Mn_{1/3}O_2$ (also referred to as $NCM_{333}$ for short), $LiNi_{0.5}Co_{0.2}Mn_{0.3}O_2$ (also referred to as $NCM_{523}$ for short), $LiNi_{0.5}Co_{0.25}Mn_{0.25}O_2$ (also referred to as $NCM_{211}$ for short), $LiNi_{0.6}Co_{0.2}Mn_{0.2}O_2$ (also referred to as $NCM_{622}$ for short), $LiNi_{0.8}Co_{0.1}Mn_{0.1}O_2$ (also referred to as $NCM_{811}$ for short), $LiNi_{0.96}Co_{0.02}Mn_{0.02}O_2$ (also referred to as $Ni_{96}$ for short), lithium nickel cobalt aluminum oxide (such as $LiNi_{0.85}Co_{0.15}Al_{0.05}O_2$), and modified compounds thereof, and the like.

Examples of the lithium-containing phosphate of the olivine structure may include, but are not limited to, at least one of lithium iron phosphate (such as $LiFePO_4$ (also referred to as LFP for short)), a composite of lithium iron phosphate and carbon, lithium manganese phosphate (such as $LiMnPO_4$), a composite of lithium manganese phosphate and carbon, lithium iron manganese phosphate, a composite of lithium iron manganese phosphate and carbon.

In some embodiments, in the sodium transition metal oxide, the transition metal can be at least one of Mn, Fe, Ni, Co, Cr, Cu, Ti, Zn, V, Zr, and Ce. The sodium transition metal oxide is for example $Na_xM_yO_2$, wherein M is one or more of Ti, V, Mn, Co, Ni, Fe, Cr, and Cu, $0<x\leq 1$, $0.5<y\leq 1.5$. In some embodiments, $Na_{0.88}Cu_{0.24}Fe_{0.29}Mn_{0.47}O_2$ may be used as the positive electrode active material.

In some embodiments, polyanionic compounds can be a class of compounds having sodium ions, transition metal ions, and tetrahedral $(YO_4)^{n-}$ anionic units. The transition metal may be at least one of Mn, Fe, Ni, Co, Cr, Cu, Ti, Zn, V, Zr, and Ce; Y may be at least one of P, S and Si; N represents the valence of $(YO_4)^{n-}$.

In some embodiment, polyanionic compounds can be a class of compounds having sodium ions, transition metal ions, and tetrahedral $(YO_4)^{n-}$ anionic units and halide anions. The transition metal may be at least one of Mn, Fe, Ni, Co, Cr, Cu, Ti, Zn, V, Zr, and Ce; Y may be at least one of P, S and Si, and n represents the valence of $(YO_4)^{n-}$; and the halogen can be at least one of F, Cl, and Br.

In some embodiments, polyanionic compounds can also be a class of compounds having sodium ions, tetrahedral $(YO_4)^{n-}$ anionic units, polyhedral units $(ZO_y)^{m+}$, and optionally halide anions. Y may be at least one of P, S and Si, and n represents the valence of $(YO_4)^{n-}$; Z represents a transition metal, which may be at least one of Mn, Fe, Ni, Co, Cr, Cu, Ti, Zn, V, Zr, and Ce, and m represents the valence of $(ZO_y)^{m+}$; and the halogen can be at least one of F, Cl, and Br.

In some embodiments, the polyanionic compound is, for example, at least one of $NaFePO_4$, $Na_3V_2(PO_4)_3$, $NaM'PO_4F$ (M' is one or more of V, Fe, Mn, and Ni), and $Na_3(VO_y)_2(PO_4)_2F_{3-2y}$ ($0\leq y\leq 1$).

In some embodiments, the Prussian blue-based compounds may be a class of compounds having a sodium ion, a transition metal ion, and a cyanide ion ($CN^-$). The transition metal can be at least one of Mn, Fe, Ni, Co, Cr, Cu, Ti, Zn, V, Zr, and Ce. Prussian blue-based compounds are for example $Na_aMe_bMe'_c(CN)_6$, wherein Me and Me' are each independently at least one of Ni, Cu, Fe, Mn, Co, and Zn, $0<a\leq 2$, $0<b<1$, $0<c<1$.

In some embodiments, the positive film layer also optionally includes a binder. As an example, the binder may include at least one of polyvinylidene fluoride (PVDF), tetrafluoroethylene (PTFE), vinylidene fluoride-tetrafluoroethylene-propylene terpolymer, vinylidene fluoride-hexafluoropropylene-tetrafluoroethylene terpolymer, tetrafluoroethylene-hexafluoropropylene copolymer, and a fluorine-containing acrylate resin.

In some embodiments, the positive film layer also optionally includes a conductive agent. As an example, the conductive agent may include at least one of superconducting carbon, acetylene black, carbon black, ketjen black, carbon dots, carbon nanotubes, graphene, and carbon nanofibers.

In some embodiments, the positive electrode plate can be prepared by: dispersing the above-mentioned components for preparing the positive electrode plate, such as a positive electrode active material, a conductive agent, a binder and any other components, in a solvent (such as N-methyl pyrrolidone) to form a positive electrode slurry; coating the the positive electrode current collector with the positive electrode slurry, and the positive electrode plate can be obtained after drying, cold pressing and other processes.

[Negative electrode plate]

The negative electrode plate includes a negative electrode current collector and a negative film layer optionally arranged on at least one surface of the negative electrode current collector, wherein the negative film layer includes a negative electrode active material.

As an example, the negative electrode current collector has two surfaces opposed in its own thickness direction, and a negative film layer (if present) is arranged on either or both of the two surfaces opposed to the negative electrode current collector.

In some embodiments, the negative electrode current collector may be a metal foil or a composite current collector. For example, a copper foil may be used as the metal foil. Optionally, the negative current collector is a metal foil, more optionally a copper foil. The composite current collector may include a polymer material substrate and a metal layer formed on at least one surface of the polymer material base layer. The composite current collector may be formed by forming a metallic material (copper, copper alloy, nickel, nickel alloy, titanium, titanium alloy, silver, silver alloy, etc.) on a substrate of a high molecular material such as a substrate of polypropylene (PP), polyethylene terephthalate (PET), polybutylene terephthalate (PBT), polystyrene (PS), polyethylene (PE), etc.

In some embodiments, the negative film layer, if present, is a lithium-containing metal or a sodium-containing metal, optionally a lithium-containing metal; optionally, the lithium-containing metal is simple lithium metal or a lithium-containing alloy. The lithium-containing alloy is an alloy formed by lithium and other metallic elements or non-metallic elements, wherein the metallic elements include tin (Sn), zinc (Zn), aluminum (Al), magnesium (Mg), silver (Ag), gold (Au), gallium (Ga), indium (In), and platinum (Pt), and combinations thereof; the non-metallic elements include boron (B), carbon (C), and silicon (Si) and combinations thereof. Optionally, the lithium-containing alloy is an alloy of lithium with other metallic elements or non-metallic elements, wherein the metallic elements include tin (Sn), zinc (Zn), aluminum (Al), magnesium (Mg), silver (Ag), gold (Au), gallium (Ga), indium (In), and platinum (Pt), and combinations thereof; the non-metallic elements include boron (B), carbon (C), and silicon (Si) and combinations thereof.

In some embodiments, the negative electrode plate includes a negative current collector and a negative film layer arranged on at least one surface of the negative current collector. The negative film layer includes lithium/sodium metal substances as negative electrode active materials. In some embodiments, the negative film layer is lithium foil.

In some embodiments, the plate is prepared by applying a lithium-containing metal foil or a sodium-containing metal foil as a negative film layer to at least one surface of a negative electrode current collector by, for example, rolling and the like to form a negative electrode plate.

[Separator]

In some embodiments, the secondary battery further includes a separator. There is no particular limitation on the kind of the separator in the present disclosure, and any known separator with a porous structure having good chemical stability and mechanical stability can be selected.

In some embodiments, the separator may use, not limited to, a polyethylene porous film, a polypropylene porous film, a polyimide porous film, and a porous film compounded by various polymers.

The separator may be a single-layer film or a multi-layer composite film, without particular limitation. When the separator is a multilayer composite film, the materials of each layer may be the same or different, without particular limitation.

In some embodiments, the positive electrode plate, the negative electrode plate, and the separator can be made into an electrode assembly by a winding process or a lamination process.

In some embodiments, the battery cell may include an outer package. The outer package may be used to encapsulate the electrode assembly and the electrolyte.

In some embodiments, the outer package of the battery cell may be a hard case such as a hard plastic case, an aluminum case, a steel case, and the like. The outer package of the battery cell may also be a soft package, such as a pouch-type soft package. The material of the soft bag may be plastic, and as the plastic, polypropylene, polybutylene terephthalate, polybutylene succinate, etc. may be listed.

The shape of the battery cell is not particularly limited in the present disclosure, and may be cylindrical, square or any other shape. For example, Fig. 1 is a battery cell 5 having a square structure as one example.

In some embodiments, referring to Fig. 2, the outer package may include a housing 51 and a cover plate 53. Wherein, the housing 51 may include a bottom plate and a side plate connected to the bottom plate, the bottom plate and the side plate are enclosed to form a accommodating cavity. The housing 51 has an opening communicating with the accommodating cavity, and the cover plate 53 may be covered on the opening to enclose the accommodating cavity. The positive electrode plate, the negative electrode plate, and the separator may form the electrode assembly 52 by a winding process or a lamination process. An electrode assembly 52 is encapsulated within the accommodating cavity. The electrolyte is infiltrated into the electrode assembly 52. The number of the electrode assemblies 52 contained in the

battery cell 5 may be one or more, and a person skilled in the art would have been able to make a selection according to specific actual requirements.

In some embodiments, the battery cell can be assembled into a battery module or a battery pack, and the number of secondary batteries contained in the battery module or battery pack can be one or more, and the specific number can be selected by a person skilled in the art according to the application and capacity of the battery module.

Fig. 3 is a battery module 4 as one example. Referring to fig. 3, in the battery module 4, a plurality of battery cells 5 may be sequentially arranged along a length direction of the battery module 4. Of course, they may be arranged in any other manner. The plurality of battery cells 5 may further be fixed via fasteners.

Optionally, the battery module 4 may further include a housing having a accommodating space in which the plurality of battery cells 5 are received.

In some embodiments, the above-mentioned battery modules can also be assembled into a battery pack, and the number of battery modules contained in the battery pack can be one or more, and the specific number can be selected by a person skilled in the art according to the application and capacity of the battery pack.

Figs. 4 and 5 are a battery pack 1 as one example. Referring to Figs. 4 and 5, a battery box and a plurality of battery modules 4 provided in the battery box may be included in the battery pack 1. The battery box includes an upper box 2 and a lower box 3. The upper box 2 can cover on the lower box 3 and forms an enclosed space for accommodating the battery module 4. The plurality of battery modules 4 may be arranged in the battery box in an arbitrary manner.

In addition, the present disclosure also provides an electrical device including at least one of the secondary battery, the battery module, or the battery pack provided herein. The secondary battery, the battery module, or the battery pack may be used as a power source for the electrical device as well as an energy storage unit for the electrical device. The electrical device may include, but not limited to, mobile equipment (e.g. cell phones, notebook computers, etc.), electric vehicles (e.g. pure electric vehicles, hybrid electric vehicles, plug-in hybrid electric vehicles, electric bicycles, electric scooters, electric golf carts, electric trucks, etc.), electric trains, ships and satellites, energy storage systems, etc.

As the electrical device, the secondary battery, the battery module, or the battery pack may be selected according to its usage requirements.

Fig. 6 is an electrical device as one example. The electrical device is a pure electric vehicle, a hybrid electric vehicle, or a plug-in hybrid electric vehicle. To meet the high power and high energy density requirements of the secondary battery for the electrical device, the battery pack or battery module may be employed.

As another example, the device may be a cell phone, tablet computer, notebook computer, etc. The device is generally required to be light and thin, and a secondary battery may be used as a power source.

## Examples

Hereinafter, examples of the present disclosure will be described. The examples described below are exemplary and are intended to be illustrative of the present disclosure and are not to be construed as limiting the present disclosure. Where specific techniques or conditions are not specified in the examples, they are performed according to techniques or conditions described in the literature or in the art or according to the product manual. The reagents or instruments used, without indicating the manufacturer, are conventional products commercially available.

## Example 1

1. Preparation of electrolyte

1.87 g of lithium bisfluorosulfonimide was taken, added into 5 ml of solvent of formula 1-1, and stirred thoroughly to form a colorless transparent solution with concentration of electrolyte salt of 1M, which was the electrolyte of the present disclosure.

2. Preparation of negative electrode plate

The lithium foil with a thickness of 50 μm was applied to one surface of a copper foil with a thickness of 12 μm by rolling, and then cut into a rectangular shape of 41 mm*51 mm to be used as a negative electrode plate.

3. Preparation of positive electrode plate

The positive electrode active material $LiNi_{0.8}Co_{0.1}Mn_{0.1}O_2(NCM_{811})$ (NMC), the conductive agent acetylene black and the binder polyvinylidene fluoride (PVDF) were mixed in a mass ratio of 98:1:1, a solvent N-methyl pyrrolidone (NMP) is added and stirred until the system was uniform to obtain a positive electrode slurry (with a solid content of 70%); the positive electrode slurry was evenly double-coated on the positive electrode current collector aluminum foil

at a loading of about 25 mg/$cm^2$, dried at room temperature, transferred to an oven for further drying, and then cut into a rectangle of 40 mm*50 mm as the positive electrode plate.

4. Separator

The polyethylene porous membrane was selected and cut into a rectangle of 45mm*55mm for future use.

5. Preparation of battery

A cut positive electrode plate was taken and matched with two cut negative electrode plates. One side of the negative electrode plate covered with lithium foil on faces the positive electrode plate, and the positive and negative electrode plates were separated by the aforementioned separator between them, and wrapped in an aluminum plastic film bag to form a laminated dry cell. 0.3g of the electrolyte prepared as described above was injected, and vacuum hot-press packaging was performed on the aluminium-plastic film bag; after standing at room temperature for at least 6 hours, the cycle test can be started. The laminated battery thus prepared had a rated capacity of 140 mAh.

6. Test of battery performance

(1) Cycling performance test of the battery cell at room temperature

The battery prepared as above was taken, charged at 25 °C at an ambient temperature using a constant current with a rate of 0.2C (namely, 28 mA) to reach a cut-off voltage of 4.3V, then charged with a constant voltage of 4.3V until the current decays to 0.1C (namely, 14mA), and then discharged at a rate of 1C (namely, 140 mA) to 2.8 V, namely, a cycle of charge and discharge. Charge-discharge cycles were performed as such, and the discharge capacity of each cycle was tested. When the discharge capacity of a certain number of cycle decays to 80% of the first cycle discharge capacity, the battery life was considered to be cut-off, while the number of cycles at this time was the cycle life. The results of the tests were shown in Table 1.

(2) Test for high and low-temperature discharge capacities of battery cells

The batteries prepared above were placed in a Vötsch VT4044 high and low temperature chamber. First, the temperature of the high and low temperature chamber was set to 25°C, and charge/discharge cycles were performed for 2 cycles in the manner described in (1) above to activate the battery cells. Then, the temperatures of high and low chambers were set to -40°C and 25°C, respectively, and charge-discharge cycles were performed for 3 cycles in the manner described in (1) above, and the discharge capacity per cycle was recorded, and the average value was calculated.

The low-temperature capacity retention rate was calculated using the following formula:

$$\text{capacity retention rate} = \frac{\text{average discharge capacity at } -40℃}{\text{average discharge capacity at } 25℃} \times 100\%.$$

The test results were shown in Table 1 below.

(3) Cycling performance test of the battery cell at low temperature

The cell prepared as above was taken and placed in Vötsch VT4044 high and low temperature test chamber, the temperature of high and low temperature chamber was set as -40°C, charge-discharge cycle was performed in the manner as described in (1) above, and the discharge capacity of each cycle was recorded. When the discharge capacity of a certain number of cycle decays to 80% of the first cycle discharge capacity, the battery life was considered to be cut-off, while the number of cycles at this time was the cycle life of the battery. The results of the tests were shown in Table 1.

**Examples 2-31**

The preparation and testing methods of Examples 2-7 were similar to those of Example 1, except that the concentration of electrolyte salt was different, as shown in Table 1.

The preparation and testing methods and the concentration of electrolyte salt of Examples 8-14 were similarly to those of Examples 1-7, respectively, except that the compound of Formula I was 1-9, as detailed in Table 1.

The preparation and testing methods and the concentration of electrolyte salt of Examples 15-21 were similarly

to those of Examples 1-7, respectively, except that the compound of Formula I was 1-57, as detailed in Table 1.

The preparation and testing methods and the concentration of electrolyte salt of Examples 22-28 were similarly to those of Examples 1-7, respectively, except that the compound of Formula I was 1-78, as detailed in Table 1.

The preparation and testing methods of Examples 29-31 were similar to those of Example 1 except that compounds of Formula I were 1-56, 1-73 and 1-143, respectively, as detailed in Table 1.

**Examples 32-33**

The preparation and testing methods of Examples 32-33 were similar to those of Example 1 except that the electrolyte salt was different, as detailed in Table 1.

**Examples 34-36**

In Examples 34-36, the preparation of the electrolyte was as follows:

First, the compound of formula 1-1 was mixed with dimethyl carbonate (DMC) in a mass ratio of 1:5 to form a homogeneous mixed solvent; 1.87g of lithium bisfluorosulfonimide salt (LiFSI) was added to 5 ml of the above-mentioned mixed solvent to form a uniform solution with a concentration of 2M, which was used as an electrolyte for subsequent tests.

The remainder of the preparation and testing methods was similar to Example 1.

**Comparative Example 1**

The preparation and testing methods of Comparative Example 1 were similar to Example 1 except that the solvent was tetrahydrofuran, as detailed in Table 1.

**Comparative Example 2**

The preparation and testing methods of Comparative Example 2 were similar to Example 1 except that the solvent was 1,3-dioxolane, as detailed in Table 1.

**Comparative Example 3**

The preparation and testing methods of Comparative Example 3 were similar to Example 1 except that the solvent was 1,3-dioxane as detailed in Table 1.

**Comparative Example 4**

The preparation and testing methods of Comparative Example 4 were similarly to Example 1, except that the electrolyte salt was weighed in an amount of 0.28 g and the final concentration was 0.3M, as detailed in Table 1.

**Comparative Example 5**

The preparation and testing methods of Comparative Example 5 were similarly to Example 1, except that the electrolyte salt was weighed in an amount of 5.61 g and the final concentration was 6M, as detailed in Table 1.

Table 1:

| Serial number | Lithium salt | Concentration of lithium salt (M) | Compound of formula I | Other solvents | Mass percentage of compounds of formula I [1] | Cycle life at 25°C (cycle) | Capacity retention rate at -40°C | Cycle life at -40°C (cycle) |
|---|---|---|---|---|---|---|---|---|
| Example 1 | LiFSI | 2 | I-1 | - | 100% | 402 | 61% | 283 |
| Example 2 | LiFSI | 0.5 | I-1 | - | 100% | 282 | 63% | 209 |
| Example 3 | LiFSI | 5 | I-1 | - | 100% | 387 | 57% | 217 |
| Example 4 | LiFSI | 1 | I-1 | - | 100% | 333 | 62% | 257 |
| Example 5 | LiFSI | 3 | I-1 | - | 100% | 389 | 60% | 249 |

(continued)

| Serial number | Lithium salt | Concentration of lithium salt (M) | Compound of formula I | Other solvents | Mass percentage of compounds of formula I [1] | Cycle life at 25°C (cycle) | Capacity retention rate at -40°C | Cycle life at -40°C (cycle) |
|---|---|---|---|---|---|---|---|---|
| Example 6 | LiFSI | 1.5 | I-1 | - | 100% | 384 | 61% | 277 |
| Example 7 | LiFSI | 2.5 | I-1 | - | 100% | 401 | 61% | 271 |
| Example 8 | LiFSI | 2 | I-9 | - | 100% | 393 | 59% | 281 |
| Example 9 | LiFSI | 0.5 | I-9 | - | 100% | 274 | 60% | 201 |
| Example 10 | LiFSI | 5 | I-9 | - | 100% | 371 | 55% | 217 |
| Example 11 | LiFSI | 1 | I-9 | - | 100% | 328 | 59% | 255 |
| Example 12 | LiFSI | 3 | I-9 | - | 100% | 380 | 57% | 238 |
| Example 13 | LiFSI | 1.5 | I-9 | - | 100% | 367 | 59% | 274 |
| Example 14 | LiFSI | 2.5 | I-9 | - | 100% | 390 | 59% | 266 |
| Example 15 | LiFSI | 2 | I-57 | - | 100% | 390 | 58% | 270 |
| Example 16 | LiFSI | 0.5 | I-57 | - | 100% | 275 | 60% | 188 |
| Example 17 | LiFSI | 5 | I-57 | - | 100% | 373 | 57% | 206 |
| Example 18 | LiFSI | 1 | I-57 | - | 100% | 322 | 60% | 234 |
| Example 19 | LiFSI | 3 | I-57 | - | 100% | 381 | 58% | 237 |
| Example 20 | LiFSI | 1.5 | I-57 | - | 100% | 364 | 58% | 260 |
| Example 21 | LiFSI | 2.5 | I-57 | - | 100% | 390 | 57% | 265 |
| Example 22 | LiFSI | 2 | I-78 | - | 100% | 364 | 51% | 215 |
| Example 23 | LiFSI | 0.5 | I-78 | - | 100% | 277 | 53% | 132 |
| Example 24 | LiFSI | 5 | I-78 | - | 100% | 332 | 46% | 155 |
| Example 25 | LiFSI | 1 | I-78 | - | 100% | 336 | 52% | 182 |
| Example 26 | LiFSI | 3 | I-78 | - | 100% | 348 | 49% | 191 |
| Example 27 | LiFSI | 1.5 | I-78 | - | 100% | 328 | 51% | 204 |
| Example 28 | LiFSI | 2.5 | I-78 | - | 100% | 355 | 51% | 210 |
| Example 29 | LiFSI | 2 | I-56 | - | 100% | 358 | 54% | 206 |
| Example 30 | LiFSI | 2 | I-73 | - | 100% | 334 | 52% | 198 |
| Example 31 | LiFSI | 2 | I-143 | - | 100% | 328 | 50% | 192 |
| Example 32 | $LiPF_6$ | 2 | I-1 | - | 100% | 337 | 54% | 208 |
| Example 33 | LiBOB | 2 | I-1 | - | 100% | 295 | 48% | 188 |
| Example 34 | LiFSI | 2 | I-1 | DMC | 20% | 311 | 50% | 187 |
| Example 35 | LiFSI | 2 | I-1 | DMC | 60% | 378 | 54% | 261 |
| Example 36 | LiFSI | 2 | I-1 | Perfluoropentanone | 60% | 384 | 62% | 267 |
| Comparative Example 1 | LiFSI | 2 | Tetrahydrofuran | - | 100% | 41 | 60% | 31 |

(continued)

| Serial number | Lithium salt | Concentration of lithium salt (M) | Compound of formula I | Other solvents | Mass percentage of compounds of formula I [1] | Cycle life at 25°C (cycle) | Capacity retention rate at -40°C | Cycle life at -40°C (cycle) |
|---|---|---|---|---|---|---|---|---|
| Comparative Example 2 | LiFSI | 2 | 1,3-dioxopentacyclo | - | 100% | 36 | 56% | 27 |
| Comparative | LiFSI | 2 | 1,3-diox | - | 10 | 34 | 49% | 19 |
| Example 3 | | | ane | | 0% | | | |
| Comparative Example 4 | LiFSI | 0.3 | I-1 | - | 100% | 96 | 63% | 57 |
| Comparative Example 5 | LiFSI | 6 | I-1 | - | 100% | 365 | 18% | 7 |

[1] The mass percent (or weight percent) is based on the total mass of solvent in the electrolyte.

By comparing each example with the comparative examples, it can be found that if the solvent molecules do not undergo fluorine substitution, and the capacity at low temperature still has a certain capacity retention ability, but the circulation capacity at room temperature and low temperature is significantly poor. In addition, compared with other lithium salts, lithium bis-fluorosulfonimide salts can promote capacity release and long cycle life at low temperature.

The electrolytes of the present disclosure can impart a longer cycle life (up to 402 cycles at room temperature) and good low-temperature performance (good capacity retention at -40 °C and longer cycle life) to a liquid lithium metal secondary battery, and thus can work in a wider temperature range (especially in a harsh environment at -40 °C). In particular, the electrolytes of the present disclosure, can still impart long cycle life to secondary batteries even at relatively low temperatures, wherein the cycle life can remain keep above 130 cycles (and in some examples, it can even reach above 280 cycles) under the environment of -40 °C and the charge-discharge conditions of 0.2C-1C.

It should be noted that the present disclosure is not limited to the above-mentioned embodiments. The above-mentioned embodiments are merely examples, and within the scope of the technical solution of the present disclosure, embodiments having substantially the same constitution as the technical idea and exerting the same function and effect are all included within the technical scope of the present disclosure. In addition, various modifications may be made to the embodiments by those skilled in the art without departing from the spirit of the present disclosure, and other embodiments that are constructed by combining some of the constituent elements of the embodiments are also included in the scope of the present disclosure.

## Claims

1. An electrolyte containing a solvent comprising at least one compound of Formula I:

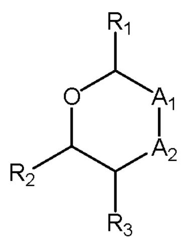

I

where

$A_1$ is an oxygen atom or a single bond;
$A_2$ is a single bond or $CHR_4$;
$R_1$ and $R_2$ are each independently a hydrogen atom, C1-C6 alkyls or C1-C6 fluoroalkyls;

$R_3$ and $R_4$ are each independently a hydrogen atom, a fluorine atom, C1-C6 alkyls or C1-C6 fluoroalkyls;
$A_1$ and $A_2$ cannot both be single bonds simultaneously, and
only one of $R_1$ to $R_4$ comprises a fluorine atom.

**2.** The electrolyte according to claim 1, wherein $R_1$ and $R_2$ are each independently a hydrogen atom, C1-C4 alkyls or C1-C4 fluoroalkyls;

optionally, $R_1$ and $R_2$ are each independently a hydrogen atom, a methyl, or a fluoromethyl;
more optionally, $R_1$ and $R_2$ are each independently a hydrogen atom or a fluoromethyl; and
still more optionally, $R_1$ and $R_2$ are each independently a hydrogen atom.

**3.** The electrolyte according to claim 1 or 2, wherein $R_3$ and $R_4$ are each independently a hydrogen atom, a fluorine atom, C1-C4 alkyls or C1-C4 fluoroalkyls; and
optionally, $R_3$ and $R_4$ are each independently a hydrogen atom, a fluorine atom, a methyl, or a fluoromethyl.

**4.** The electrolyte according to any one of claims 1 to 3, wherein $R_3$ is a fluorine atom or a hydrogen atom, optionally a fluorine atom.

**5.** The electrolyte according to any one of claims 1 to 4, wherein $R_4$ is a hydrogen atom.

**6.** The electrolyte according to any one of claims 1 to 5, wherein the compound of formula I is selected from at least one of the following compounds:

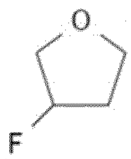
I-1
,
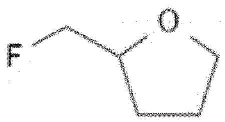
I-9
,
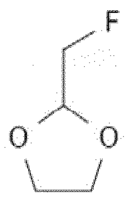
I-57
, and
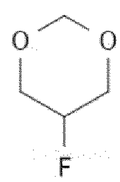
I-78
.

**7.** The electrolyte according to any one of claims 1 to 6, wherein the solvent further comprises one or more selected from: ethylene glycol dimethyl ether, ethylene glycol methyl ethyl ether, ethylene glycol diethyl ether, ethylene glycol dipropyl ether, diethylene glycol dimethyl ether, triethylene glycol dimethyl ether, tetraethylene glycol dimethyl ether, propylene glycol dimethyl ether, butylene glycol dimethyl ether, dimethoxymethane, diethoxymethane, trimethyl orthoformate, triethyl orthoformate, trimethyl orthocarbonate, triethyl orthocarbonate, ethylene carbonate, vinylene carbonate, dimethyl carbonate, methyl ethyl carbonate, diethyl carbonate, fluoroethylene carbonate, acetonitrile, dimethyl sulfoxide, sulfolane, bis(2,2,2-trifluoroethyl) ether, 1,1,2,2-tetrafluoroethyl-2,2,3,3-tetrafluoropropyl ether, 1,2-bis(1,1,2,2-tetrafluoroethoxy) ethane, fluorobenzene, p-difluorobenzene, o-difluorobenzene, m-difluorobenzene, trifluorobenzene, trifluorotoluene, trifluoromethoxybenzene, acetone and perfluoropentanone; optionally, the solvent further comprises dimethyl carbonate and/or perfluoropentanone.

**8.** The electrolyte according to any one of claims 1 to 7, wherein the solvent comprises at least 20 wt%, more optionally at least 60 wt% of the compound of formula I, based on a total weight of the solvent.

**9.** The electrolyte according to any one of claims 1 to 8, wherein the electrolyte further comprises an additive selected from at least one of: succinic anhydride, vinylene carbonate, 1,3-propane sultone, ethylene vinyl carbonate, ethylene fluorocarbonate, vinyl sulfite, tris(trimethylsilane)phosphate.

**10.** The electrolyte according to any one of claims 1 to 9, wherein a content of the additive is 5wt% or less based on the total weight of the electrolyte.

**11.** The electrolyte according to any one of claim 1 to 10, wherein the electrolyte further comprises an electrolyte salt selected from at least one of lithium bis-fluorosulfonimide, lithium hexafluorophosphate, lithium tetrafluoroborate, lithium hexafluoroarsenate, lithium bis-trifluoromethane sulfonimide, lithium triflate, lithium difluorophosphate, lithium bis(oxalato) borate, lithium difluorooxalato borate, lithium difluoro bis(oxalato) phosphate, and lithium

tetrafluorooxalato phosphate; optionally, at least one of lithium bis-fluorosulfonimide, lithium hexafluorophosphate, and lithium tetrafluoroborate; more optionally, lithium bis-fluorosulfonimide.

**12.** The electrolyte according to any one of claim 1 to 11, wherein a concentration of the electrolyte salt is 0.5 M to 5 M, optionally 1 M to 3 M, more optionally 1.5 M to 2.5 M.

**13.** A battery cell comprising the electrolyte according to any one of claims 1 to 12.

**14.** The battery cell according to claim 13, wherein the battery cell further comprises a negative electrode plate comprising a current collector and optionally a layer of negative material disposed on at least one surface of the current collector, wherein the negative material is a lithium-containing metal; optionally, the lithium-containing metal is simple lithium metal or a lithium-containing alloy; optionally, the lithium-containing alloy is an alloy formed by lithium and other metallic elements or non-metallic elements, wherein the metallic elements comprise tin (Sn), zinc (Zn), aluminum (Al), magnesium (Mg), silver (Ag), gold (Au), gallium (Ga), indium (In), and platinum (Pt), and combinations thereof; the non-metallic elements include boron (B), carbon (C), and silicon (Si) and combinations thereof.

**15.** A battery comprising the battery cell according to claim 13 or 14.

**16.** An electrical device comprising the battery cell according to claim 13 or 14 and/or the battery according to claim 15.

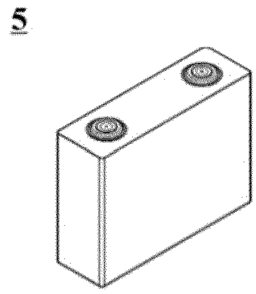


FIG. 1

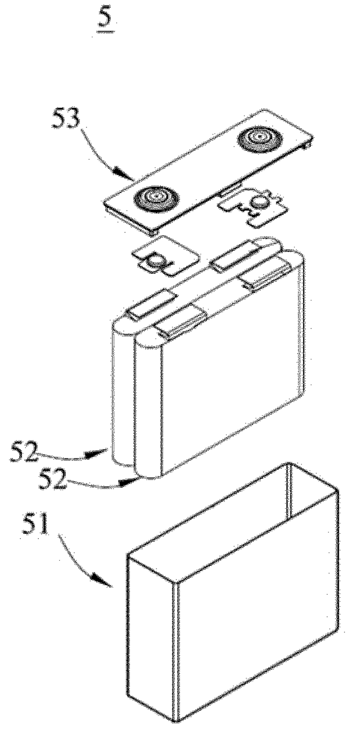


FIG. 2

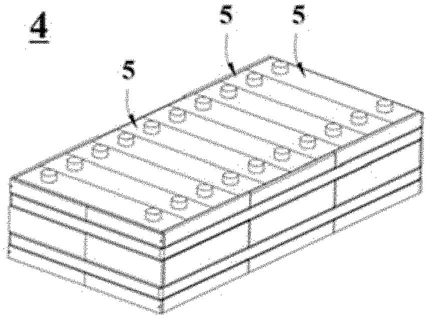


FIG. 3

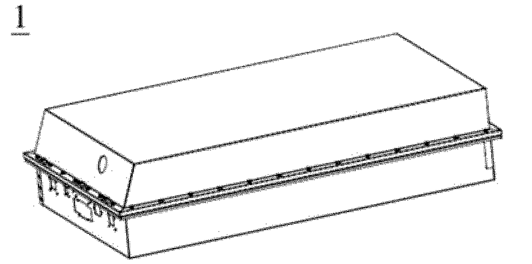


FIG. 4

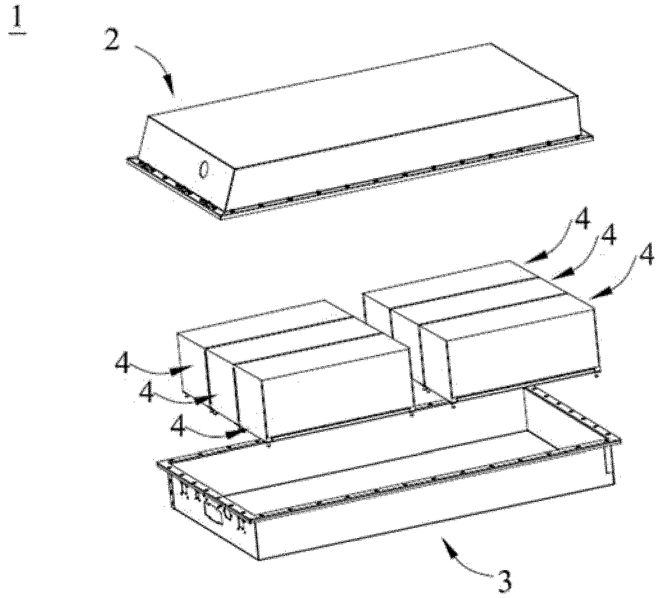


FIG. 5

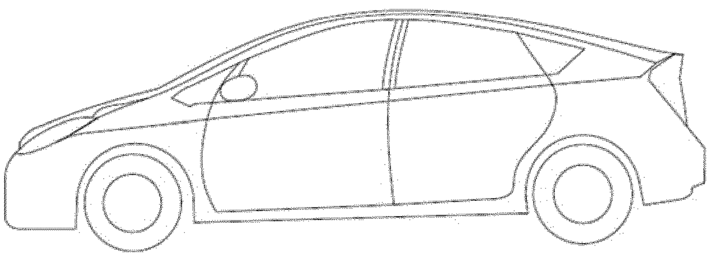

FIG. 6

**INTERNATIONAL SEARCH REPORT**

International application No.
**PCT/CN2022/125067**

**A. CLASSIFICATION OF SUBJECT MATTER**

H01M10/0569(2010.01)i

According to International Patent Classification (IPC) or to both national classification and IPC

**B. FIELDS SEARCHED**

Minimum documentation searched (classification system followed by classification symbols)

IPC: H01M

Documentation searched other than minimum documentation to the extent that such documents are included in the fields searched

Electronic data base consulted during the international search (name of data base and, where practicable, search terms used)

CNTXT; ENTXT; ENTXTC; DWPI; CNKI: 锂, 电池, 电解液, 溶剂, 锂盐, 添加剂, 氟, 代, 呋喃, 氧戊环, 二氧戊烷, lithium, battery, electrolyte, solution, solvent, salt, additive, fluorine, generation, furan, oxolane, dioxolane

**C. DOCUMENTS CONSIDERED TO BE RELEVANT**

| Category* | Citation of document, with indication, where appropriate, of the relevant passages | Relevant to claim No. |
|---|---|---|
| X | CN 102479977 A (ZHANGJIAGANG GUOTAI-HUARONG NEW CHEMICAL MATERIALS CO., LTD.) 30 May 2012 (2012-05-30)<br>description, paragraphs 9-29 | 1-16 |
| X | CN 114555575 A (MEXICHEM FLUOR, S.A. DE C.V.) 27 May 2022 (2022-05-27)<br>description, paragraphs 73-149 | 1-16 |
| X | JP 2003297417 A (MITSUBISHI CHEMICAL CO., LTD. et al.) 17 October 2003 (2003-10-17)<br>description, paragraphs 4-36 | 1-16 |
| A | CN 101154753 A (SANYO ELECTRIC CO., LTD.) 02 April 2008 (2008-04-02)<br>entire document | 1-16 |
| A | US 5750730 A (SANYO CHEMICAL INDUSTRIES, LTD.) 12 May 1998 (1998-05-12)<br>entire document | 1-16 |

☐ Further documents are listed in the continuation of Box C.  ☑ See patent family annex.

* Special categories of cited documents:

"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 April 2023** | **09 May 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2022/125067**

| Patent document cited in search report | | | Publication date (day/month/year) | Patent family member(s) | | | Publication date (day/month/year) |
|---|---|---|---|---|---|---|---|
| CN | 102479977 | A | 30 May 2012 | None | | | |
| CN | 114555575 | A | 27 May 2022 | EP | 4042507 | A1 | 17 August 2022 |
| | | | | WO | 2021069923 | A1 | 15 April 2021 |
| | | | | GB | 202006716 | D0 | 17 June 2020 |
| | | | | KR | 20220078599 | A | 10 June 2022 |
| | | | | TW | 202122378 | A | 16 June 2021 |
| | | | | GB | 201916374 | D0 | 25 December 2019 |
| | | | | JP | 2022551173 | A | 07 December 2022 |
| JP | 2003297417 | A | 17 October 2003 | None | | | |
| CN | 101154753 | A | 02 April 2008 | JP | 2008084705 | A | 10 April 2008 |
| | | | | JP | 5094084 | B2 | 12 December 2012 |
| | | | | US | 2008081261 | A1 | 03 April 2008 |
| | | | | US | 8097367 | B2 | 17 January 2012 |
| US | 5750730 | A | 12 May 1998 | DE | 19700656 | A1 | 24 July 1997 |
| | | | | DE | 19700656 | B4 | 31 August 2006 |

Form PCT/ISA/210 (patent family annex) (July 2022)